(19) Europäisches Patentamt
European Patent Office
Office européen des brevets

(11) Veröffentlichungsnummer : **0 110 814 B1**

(12) # EUROPÄISCHE PATENTSCHRIFT

(45) Veröffentlichungstag der Patentschrift : **13.12.89**

(21) Anmeldenummer : **83730103.5**

(22) Anmeldetag : **27.10.83**

(51) Int. Cl.[4] : **C 07 D471/04**, C 07 F 9/65, A 61 K 31/44, A 61 K 31/675// C07D209/10, C07D209/14, C07D209/16 ,(C07D471/04, 221:00, 209:00)

(54) **Beta-Carboline, Verfahren zu ihrer Herstellung und diese enthaltende pharmazeutische Präparate.**

(30) Priorität : **29.10.82 DE 3240514**

(43) Veröffentlichungstag der Anmeldung : **13.06.84 Patentblatt 84/24**

(45) Bekanntmachung des Hinweises auf die Patenterteilung : **13.12.89 Patentblatt 89/50**

(84) Benannte Vertragsstaaten : **AT BE CH DE FR GB IT LI LU NL SE**

(56) Entgegenhaltungen :
**EP–A– 0 054 507**
**JOURNAL OF MEDICINAL CHEMISTRY, Band 25, Nr. 9, Seiten 1081-1091,1982, American Chemical Society, Washington, US M.CAIN: Beta-Carbolines: Synthesis and Neurochemical and Pharmacological Actions on Brain Benzodiazepine Receptors**
**JOURNAL OF PHARMACEUTICAL SCIENCES, Band 59, Nr. 10, Seiten 1445-1448, Oktober 1970, Washington, US BENG T. HO et al.: "Inhibitors of Monoamine Oxidase VI: Effects of Substitution on Inhibitory Activity of 6(or 8)-Substituted Beta-Carbolines**
**JOURNAL OF PHARMACEUTICAL SCIENCES; Band 58, Nr, 2, Seiten 219-221, Februar 1969, Washington, US BENG T. HO etal: "Inhibitors of Monoamine Oxidase III: 9-Substituted-Beta-Carbolines "**
**CEMICAL ABSTRACTS, Band 77, Seite 445, 1972, Zusammenfassung 140008y, Columbus, Ohio, US & JP - A - 7230718**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Huth, Andreas, Dr.**
**Kyllmannstrasse 15**
**D-1000 Berlin 39 (DE)**
Erfinder : **Rahtz, Dieter, Dr.**
**Krottnaurer Strasse 24a**
**D-1000 Berlin 38 (DE)**
Erfinder : **Seidelmann, Dieter, Dr.**
**Stierstrasse 14**
**D-1000 Berlin 41 (DE)**
Erfinder : **Schmiechen, Ralph, Dr.**
**Bayernring 27**
**D-1000 Berlin 42 (DE)**
Erfinder : **Biere, Helmut, Dr.**
**Zeltinger Strasse 15**
**D-1000 Berlin 28 (DE)**
Erfinder : **Braestrup, Claus Thyco, Dr.**
**Frederiksborgvej 78**
**DK-4000 Roskilde (DK)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

CHEMICAL ABSTRACTS, Band 58, Spalte 12842, 1963, Zusammenfassung 12842c, Columbus, Ohio, US Y.C.TUNG et al.: "Beta-Carboline derivatives. I. Synthesis of 3-methylnorharman derivatives and their effects on monoamine oxidase and choline oxidase"

CHEMICAL ABSTRACTS , Band 55, Spalte 22314, 1961, Zusammenfassung 22314b, Columbus, Ohio, US I.CHIH TUNG: "Beta-Carboline derivatives. II. Synthesis of norharman, harman, and 3-methyl-harman derivatives and their effects on monoamine oxidase choline oxidase, and succinic oxidase "

CHEMICAL ABSTRACTS, Band 84, Seite 192, 1976, Zusammemfassung 131965a, Columbus, Ohio, US R.D. STEBBINS et al.: "Indentification of N5,N10-methylene tetrahydrofolate reductase as the enzyme involved in the 5-methyl tetrahydrofolate-dependent formation of a beta-carboline derivative of 5-hydroxytryptamine in human platelets"

BULLETIN DE LA SOCIETE CHIMIQUE DE FRANCE, Seite 82, 1975, Paris, FR B.P. ROQUES et al.:"Nr, 447 - Détermination de la structure de la bromonorharmane par effet Overhauser"

JOURNAL OF THE AMERICAN CHEMICAL SOCIETY, Band 87:18, September 20, 1965, Seiten 4168-4174, Washington, US E. LEETE et al.:"Biosynthesis of the Vinca Alkaloids. I. Feeding Experiments with Tryptophan-2C14 and Acetate-1-C14"

JOURNAL OF ORGANIC CHEMISTRY, Band 21, Seiten 295-296, Washington, US R.C. ELDERFIELD et al: "Alstonia Alkaloids. VI. The Structure of Alstoniline Oxide"

CHEMICAL ABSTRACTS, Band 65, Spalte 2239, 1966, Zusammenfassung 2239d, Columbus, Ohio, US J.P. SAXENA: "Mononitration of alpha- and beta-carbolines"

Die Akte enthält technische Angaben, die nach dem Eingang der Anmeldung eingereicht wurden und die nicht in dieser Patentschrift enthalten sind.

**Beschreibung**

Die Erfindung betrifft β-Carboline, die in 3-Stellung einen Substituenten enthalten, der sich nicht von einer Carbonsäure ableitet. Bisher ist man davon ausgegangen, daß das Vorliegen einer 3-Carbonsäure oder eines Derivats einer 3-Carbonsäure für die Wirkung der β-Carboline ausschlaggebend ist, obwohl aus C.A. Vol. 88 (1972) Nr. 140008 und aus J. Med. Chem. 1982, Vol. 25, Nr. 9, 1081-1091 pharmazeutisch wirksame 3-Alkyl-β-carbolin-Derivate bekannt sind.

Demgegenüber wurde nun gefunden, daß die neuen β-Carboline der allgemeinen Formel I die gleichen günstigen Eigenschaften auf das Zentralnervensystem ausüben wie die bekannten in 3-Stellung durch einen carboxylhaltigen oder von Carboxyl abgeleiteten Rest substituierten Carboline, daß die neuen β-Carboline aber den Vorteil haben, daß ihre Wirkung länger anhält.

Die Erfindung betrifft neue β-Carboline gemäß den Ansprüchen 1-5, pharmazeutische Präparate mit den neuen β-Carbolinen gemäß Anspruch 6 und Verfahren zur Herstellung der neuen β-Carboline gemäß Anspruch 7.

Die erfindungsgemäßen β-Carboline haben die allgemeine Formel I

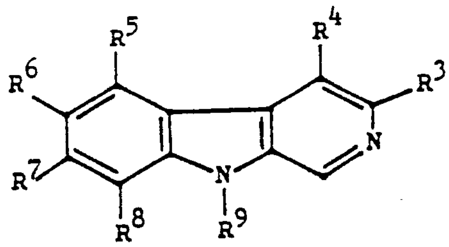

(I)

worin

$R^3$, $OR^I$ mit $R^I$ in der Bedeutung von H, $C_{1-5}$-Alkyl, $C_{3-7}$-Cycloalkyl, $C_{7-10}$-Aralkyl und $C_{5-10}$-Aryl $NR^{II}R^{III}$ mit $R^{II}$ in der Bedeutung von H, $C_{1-5}$-Alkyl, $C_{3-7}$-Cycloalkyl, $C_{7-10}$-Aralkyl und $C_{5-10}$-Aryl und $R^{III}$ in der Bedeutung von $C_{1-3}$-Acyl, ($C_{1-6}$-Alkoxy)-carbonyl, Carbamoyl und $R^{II}$ und $R^{III}$ gemeinsam mit dem N-Atom ein gesättigter oder ungesättigter 5- oder 6-Ring, $SO_nR^I$, wobei n eine Zahl zwischen 0 und 2 ist und $R^I$ die oben angegebene Bedeutung hat, $PO_3R^{IV}R^V$ mit $R^{IV}$ und $R^V$ in der Bedeutung von H, $C_{1-5}$-Alkyl, $C_{3-7}$-Cycloalkyl, $C_{7-10}$-Aralkyl und $C_{5-10}$-Aryl, wobei $R^{IV}$ und $R^V$ gleich oder verschieden sein können,

$R^4$ H, $C_{1-5}$-Alkyl, Alkoxyalkyl, $COR^{VI}$ mit $R^{VI}$ in der Bedeutung von H, $C_{1-5}$-Alkyl, $C_{3-7}$-Cycloalkyl, $C_{7-10}$-Aralkyl, OH, O-$C_{1-5}$-Alkyl, O-$C_{3-7}$-Cycloalkyl, O-$C_{7-10}$-Aralkyl, $NR^{II}R^{II}$, wobei $R^{II}$ die oben angegebene Bedeutung hat und gleich oder verschieden sein können und mit dem N-Atom einen 5- oder 6-Ring bilden können, $CSR^{VII}$ mit $R^{VII}$ in der Bedeutung von H, $C_{1-5}$-Alkyl, $C_{3-7}$-Cycloalkyl und $C_{7-10}$-Aralkyl,

$R^5$-$R^8$ H, Halogen, $NO_2$, $OR^I$, $NR^{II}R^{III}$, $PO_3R^{IV}R^V$, $SO_2NR^IR^V$, $COOR^I$, $CONR^{II}R^{III}$, $CSNR^{II}R^{III}$ und $COR^I$, wobei $R^{I-V}$ die oben angegebene Bedeutung haben und

$R^9$ H, $C_{1-5}$-Alkyl, $C_{1-3}$-Acyl, Carbamoyl, ($C_{1-6}$-Alkoxy)-carbonyl und $SO_2R^{VIII}$ mit $R^{VIII}$ in der Bedeutung von Methyl und p-Tolyl, bedeuten,

wobei der aromatische Ring mono-oder disubstituiert sein kann.

Die Substituenten in Formel I haben verschiedenartige Bedeutungen.

Unter Halogen sind im Rahmen der vorliegenden Erfindung, wenn nichts anderes vermerkt ist, Fluor, Chlor, Brom und Jod zu verstehen.

Unter Alkyl für sich oder in Verbindung mit O, S, N, CON, CSN oder $PO_3$ sind gerad- und verzweigtkettige Gruppen mit bis zu 5 Kohlenstoffatomen zu verstehen.

Cycloalkyl soll Gruppen mit 3 bis 7 Kohlenstoffatomen, Aryl mit 5 bis 10 Kohlenstoffatomen, beispielsweise Phenyl, Aralkyl mit 7 bis 10 Kohlenstoffatomen, beispielsweise Benzyl oder Styryl, einschließen.

Der aromatische Ring kann in den Stellungen 5, 6, 7 und 8 mono- oder disubstituiert sein, wobei die Substitution in 5- und 6-Stellung bevorzugt ist.

Die neuen Verbindungen der allgemeinen Formel I besitzen wertvolle pharmakologische Eigenschaften. Insbesondere wirken sie auf das Zentralnervensystem und sind somit als Psychopharmaka in der Humanmedizin geeignet.

Die erfindungsgemäßen Verbindungen können zur Formulierung von pharmazeutischen Präparationen, z. B. für die orale und parenterale Anwendung bei Säugetieren einschließlich Menschen, nach an sich bekannten Methoden der Galenik verwendet werden.

Als Hilfsstoffe zur Formulierung von pharmazeutischen Präparationen sind solche physiologisch verträglichen organischen und anorganischen Trägersubstanzen für die enterale und parenterale Anwendung geeignet, die gegenüber den erfindungsgemäßen Verbindungen inert sind.

Als Trägersubstanzen seien beispielsweise genannt Wasser, Salzlösungen, Alkohole, Polyaethylenglykole, polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermittel, Konservierungsstoffen, Stabilisatoren, Netzmittel, Emulgatoren, Puffermittel und Farbstoffen, versetzt werden.

Für die parenterale Anwendung sind insbesondere Injektionslösungen oder Suspensionen, insbesondere wässerige Lösungen der aktiven Verbindungen in polyhydroxyethoxyliertem Rizinusöl, geeignet.

Für die orale Anwendung sind insbesondere Tabletten, Dragees oder Kapseln mit Talkum und/oder einem Kohlenwasserstoffträger oder -binder, wie z. B. Lactose, Mais-oder Kartoffelstärke, geeignet. Die Anwendung kann auch in flüssiger Form erfolgen, wie z. B. als Saft, dem gegebenenfalls ein Süßstoff beigefügt wird.

Die erfindungsgemäßen Verbindungen werden in einer Dosiseinheit von 0,05 bis 10 mg aktiver Substanz in einem physiologisch verträglichen Träger eingebracht.

Die erfindungsgemäßen Verbindungen werden in einer Dosis von 0,1 bis 300 mg/Tag, vorzugsweise 1-30 mg/Tag, angewendet.

Es ist bekannt, daß bestimmte Stellen im zentralen Nervensystem von Vertebraten eine hohe spezifische Affinität für die Bindung von 1.4- und 1.5-Benzodiazepinen aufweisen (Squires, R.F. und Braestrup, C., Nature (London) 266 (1977), 734). Diese Stellen werden Benzodiazepin-Rezeptoren genannt.

Die pharmakologischen Eigenschaften der erfindungsgemäßen Verbindungen wurden durch Untersuchung des Verdrängungsvermögens von radioaktiv-markiertem Flunitrazepam von solchen Benzodiazepin-Rezeptoren bestimmt.

Die Verdrängungsaktivität der erfindungsgemäßen Verbindungen wurde bestimmt mit Hilfe des $IC_{50}$- und des $ED_{50}$-Wertes. Der $IC_{50}$-Wert gibt die Konzentration an, die eine 50 %ige Verdrängung der spezifischen Bindung von $^3$H-Flunitrazepam (1,0 nM, 0 °C) in Proben mit einem Gesamtvolumen von 0,55 ml einer Suspension von Hirnmembran, z. B. von Ratten bewirkt.

Der Verdrängungstest wird wie folgt ausgeführt:

0,5 ml einer Suspension von unbehandeltem Ratten-Haupthirn in 25 mM $KH_2PO_4$, pH = 7,1 (5-10 mg Gewebe/Probe) wird für 40-60 Minuten bei 0 °C zusammen mit $^3$H-Diazepam (spezifische Aktivität 14,4 Ci/mmol, 1,9 nM) oder $^3$H-Flunitrazepam (spezifische Aktivität 87 Ci/mmol, 1,0 nM) inkubiert. Nach Inkubation wird die Suspension durch eine Glasfritte filtriert, der Rückstand 2 mal mit kalter Pufferlösung gewaschen und die Radioaktivität am Scintillationszähler gemessen.

Der Versuch wird dann wiederholt, jedoch so, daß vor der Zugabe des radioaktiv-markiertem Benzodiazepins eine bestimmte Menge oder eine überschüssige Menge der Verbindung, deren Verdrängungsaktivität zu bestimmen ist, zugesetzt wird. Auf Grundlage der erhaltenen Werte kann dann der $IC_{50}$-Wert berechnet werden.

Der $ED_{50}$-Wert stellt die Dosis einer Versuchssubstanz dar, die eine Reduktion der spezifischen Bindung des Flunitrazepams an dem Benzodiazepin-Rezeptor in einem lebenden Gehirn auf 50 % des Kontrollwertes bewirkt.

Der in-vivo Test wird wie folgt ausgeführt:

Gruppen von Mäusen wird die Versuchssubstanz bei unterschiedlichen Dosen und normalerweise subcutan injiziert. Nach 15 Minuten wird den Mäusen das $^3$H-Flunitrazepam intravenös verabreicht. Nach weiteren 20 Minuten werden die Mäuse getötet, ihre Vorderhirnhäute entfernt und die Radioaktivität der Vorderhirnhäute durch Scintillationszählung gemessen. Der $ED_{50}$-Wert wird aus den Dosis/Wirkungs-Kurven bestimmt.

Die erfindungsgemäßen Verbindungen zeigen an Mäusen eine anti-aggressive Wirkung. Die Aggressionshemmung wurde an männlichen Mäusen (NMR von Møllegaard, DK) mit einem Gewicht von 20-22 g bestimmt. Die Mäuse werden drei Wochen lang in Plastikkäfigen isoliert gehalten, und wenn dann zwei Mäuse in den selben Käfig gebracht werden, werden sie spontan und fast sofort damit beginnen, miteinander zu kämpfen. Diese Aggression wird wirksam mit einer Reihe von psychopharmakologisch wirksamen Substanzen, einschließlich Benzodiazepinen, gehemmt (Valcelli, Mod. Probl. Pharmacopsych., 1979, 14, 143-156).

Die erfindungsgemäßen Verbindungen hemmen die Aggressionen in einem von Buus Lassen, Europ. J. Pharmacol., 1978, 47, 45-49, beschriebenen Test total. Die erfindungsgemäßen Verbindungen wurden hierzu subcutan und oral appliziert und die anti-aggressive Wirkung nach einer halben Stunde bestimmt.

Die erfindungsgemäßen Verbindungen können nach an sich bekannten Methoden hergestellt werden, dadurch daß man

a) eine Verbindung der Formel IV

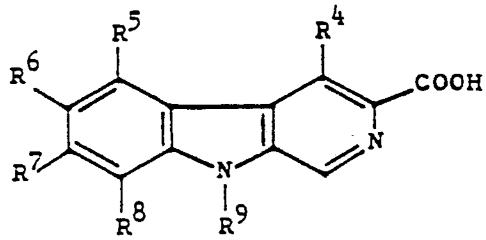

(IV)

worin $R^4$, $R^5$, $R^6$, $R^7$, $R^8$ oder $R^9$ die in Formel I angegebene Bedeutung haben, in an sich bekannter Weise zum 3-Carbonsäureazid umsetzt und das Carbonsäureazid nach Curtius in Gegenwart eines Alkohols der Formel $R^IOH$ thermisch zersetzt ;

b) ein nach a) über 3-Carbonsäureazid durch Curtiusschen Abbau erhaltenes 3-Amino-β-carbolin der Formel VI

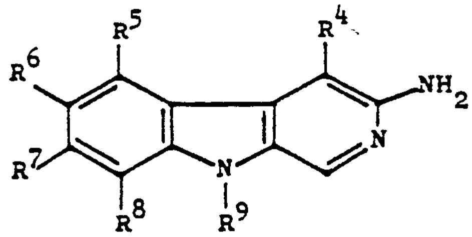

(VI)

worin $R^4$, $R^5$, $R^6$, $R^7$, $R^8$ und $R^9$ die oben angegebene Bedeutung haben, in an sich bekannter Weise acyliert, mit 2.5-Dimethoxy-tetrahydrofuran, 1.4-Dibrombutan oder 1.5-Dibrompentan umsetzt oder in einer Sandmeyer-Reaktion mit verdünnter Schwefelsäure, mit Halogenwasserstoff in Gegenwart von Kupfer (I)-halogenid, mit Alkalimetallcyanid, mit $R^IOH$ oder $(R^I)_2S$, wobei $R^I$ die oben angegebene Bedeutung besitzt, umsetzt und gewünschtenfalls eine mit $(R^I)_2S$ erhaltene 3-$SR^I$-Verbindung zur 3-$SOR^I$- oder 3-$SO_2R^I$-Verbindung oxydiert und gewünschtenfalls die 3-$SR^I$- oder 3-$SOR^I$-Gruppe mit Raney-Nickel/Wasserstoff eliminiert und gegebenenfalls anschließend in 6-Stellung halogeniert und das so erhaltene Halogenierungsprodukt gegebenenfalls zur 6-$OR^I$- oder 6-$COOR^I$-Verbindung umsetzt und gegebenenfalls die 6-$COOR^I$-Verbindung umestert, verseift oder amidiert oder in 6-Stellung nitriert und gegebenenfalls die erhaltenen Nitroverbindungen zu den entsprechenden Aminoverbindungen reduziert und gegebenenfalls die Aminoverbindungen mit Alkyl halogenid umsetzt oder in 6-Stellung sulfonyliert und gegebenenfalls die so erhaltenen Chlorsulfonylverbindungen mit einem Amin umsetzt ;

c) ein Indol der allgemeinen Formel X

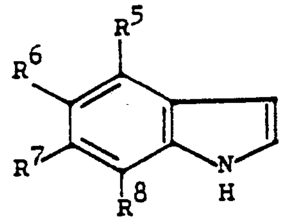

(X)

worin $R^5$, $R^6$, $R^7$ und $R^8$ die oben angegebene Bedeutung haben, mit einem Azadien der allgemeinen Formel XI

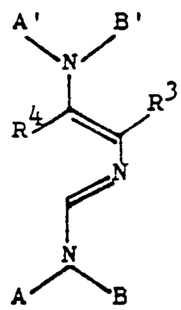

(XI)

worin $R^3$ und $R^4$ die oben angegebene Bedeutung haben und A und B sowie A′ und B′ jeweils für sich Alkyl mit 1-3 C-Atomen oder gemeinsam unter Einschluß von N eine Pyrrolidino-, Piperidino-, Morpholino- oder Piperazinogruppe bedeuten, in Gegenwart von Säuren bei Temperaturen von 50-200 °C umsetzt und gegebenenfalls einen in 3-Stellung erhaltenen Phosphonsäuremonoester in den Phosphonsäurediester überführt.

In 9-Stellung des β-Carbolins kann mit p-Toluolsulfochlorid im basischen Milieu eine Tosylgruppe oder mit N.N-Dialkylformamid-dialkylacetal in Gegenwart einer starken Base eine Alkylgruppe eingeführt werden. Als starke Basen seien beispielsweise genannt : Diazabicyclooctan, Diazabicyclononen, Diazabicycloundecen, aber auch Ethyldiisopropylamin, Kalium-tert.-butylat, Kaliumcarbonat und gepulvertes

Kaliumhydroxid. Zur Ausbildung des basischen Milieus dienen zum Beispiel Pyridin, 4-Dimethylaminopyridin, Triethylamin und Gemische aus diesen Basen.

Zum Abbau der Carboxylgruppe und zur gleichzeitigen Einführung einer 3-Alkoxycarbonylaminogruppe (Urethangruppe) gemäß Verfahren a) wird die 3-Carbonsäure zunächst in an sich bekannter Weise in das 3-Carbonsäureazid überführt. Dieses geschieht vorzugsweise durch Umsetzung der Carbonsäure mit Diphenylphosphorylazid. Das Carbonsäureazid wird anschließend dem Curtiusschen Abbau unterworfen (Organic Reactions III) (1946) 337), wobei in Gegenwart von Alkoholen die gewünschten Urethane entstehen.

Die Umsetzung der 3-Carbonsäuren zu den 3-Alkoxycarbonylamino-Verbindungen kann auch ohne die Isolierung des Säureazids als Eintopfverfahren durchgeführt werden. Nach einer bevorzugten Ausführungsform wird die in Dimethylsulfoxid gelöste β-Carbolin-3-carbonsäure zur Lösung von Diphenylphosphorylazid in Alkohol unter Zugabe von Triethylamin gegeben und unter Rückfluß erhitzt.

Die Oxydation der nach Verfahrensvariante b) erhaltenen Thioverbindungen 3-$SR^I$ zu 3-$SOR^I$ oder 3-$SO_2R^I$) erfolgt in an sich bekannter Weise. Geeignete Oxydationsmittel sind beispielsweise organische Persäuren, wie Perameisensäure, Peressigsäure, Perbenzoesäure, m-Chlorperbenzoesäure, Monoperphthalsäure, oder anorganische Peroxide, wie Wasserstoffperoxid, gelöst in Wasser oder in verdünnten organischen Säuren, anorganische Oxydationsmittel, wie Chromsäure, Salpetersäure, Chlor, Brom, Halogensauerstoffsäuren, wie unterchlorige, chlorige, Chlor- oder Jodsäure, tert.-Butylhypochlorit, organische N-Halogenverbindungen, wie N-Chlor- und N-Bromsuccinimid. Durch literaturbekannte Wahl der Reaktionsbedingungen kann das Oxydationspotential entsprechend eingestellt werden und die Reaktion zur Herstellung der Sulfoxide oder Sulfone gelenkt werden.

Nach Verfahrensvariante b) werden aus 3-Carbonsäure über 3-Carbonsäureazid durch Curtiusschen Abbau erhaltene 3-Aminoverbindungen der Formel VI in an sich bekannter Weise in 3-Stellung zum Beispiel acyliert oder mit 2.5-Dimethoxy-tetrahydrofuran, 1.4-Dibrombutan oder 1.5-Dibrompentan umgesetzt.

Die Umwandlung der 3-Aminoverbindungen in die entsprechenden 3-Hydroxy-, 3-$OR^I$- und 3-$SR^I$-Verbindungen wird ebenfalls nach bekannten Methoden durchgeführt, beispielsweise nach der Sandmeyer-Reaktion, bei der das diazotierte Produkt direkt mit verdünnter Schwefelsäure, Halogenwasserstoffsäure in Gegenwart von Kupfer(I)-halogenid, $R^IOH$ bzw. $(R^I)_2S$ bei angehobenen Temperaturen umgesetzt wird. 3-$SR^I$-Verbindungen können anschließend zu 3-$SOR^I$- und 3-$SO_2R^I$-Verbindungen oxydiert werden.

Die nach b) erhaltenen Verbindungen können anschließend zum Beispiel in 6-Stellung halogeniert, nitriert oder sulfoniert werden. In 6-Stellung halogenierte Verbindungen können gegebenenfalls anschließend alkoxyliert, thioalkyliert, carboxyliert oder mit einem Amin zur Reaktion gebracht werden.

Die Halogenierung in 6-Stellung erfolgt nach an sich bekannten Methoden. Hierzu wird das Ausgangsmaterial in einem inerten Lösungsmittel gelöst und mit dem entsprechenden Halogen wie Chlor oder Brom gegebenenfalls in Gegenwart eines basischen Katalysators bei Temperaturen unterhalb Raumtemperatur umgesetzt. Inerte Lösungsmittel sind beispielsweise chlorierte Kohlenwasserstoffe wie Methylenchlorid, Chloroform, Dichloräthylen usw. Als basische Katalysatoren eignen sich Pyridin und substituierte Pyridine wie 4-Dimethylaminopyridin. Ein basischer Katalysator ist bei der Chlorierung entbehrlich.

Zur Einführung von Jod verwendet man zweckmäßigerweise nicht nur elementares Jod, sondern ein Gemisch von Jod und Jodsäure, wobei die Reaktion vorzugsweise in Eisessig bei 80 °C unter Protonenkatalyse durchgeführt wird.

Die sich gegebenenfalls anschließende Alkoxylierung und die Thioalkylierung der Halogenverbindungen erfolgt ebenfalls nach an sich bekannten Methoden.

Die 6-Halogenverbindung, insbesondere 6-Jodverbindung, kann zum Beispiel mit Palladium (II) acetat und Kohlenmonoxid in Benzylalkohol und in Gegenwart eines tert. Amins, wie zum Beispiel Triethylamin, Tributylamin oder Pyridin, carbonyliert werden, Die so erhaltene 6-Benzyloxycarbonylverbindung kann anschließend in bekannter Weise verseift, umgeestert oder mit einem Amin zur Reaktion gebracht werden.

Zur Umesterung wird der vorliegende Ester mit einem Alkohol $R^IOH$ in Gegenwart katalytischer Mengen von $R^IONa$ oder NaH 3-6 Stunden auf Temperaturen zwischen 60 und 120 °C erhitzt. Gegebenenfalls kann die Umesterung mit dem Alkohol $R^IOH$ auch in Gegenwart eines sauren Katalysators wie p-Toluolsulfonsäure, HCl oder $CuCl_2$ vorgenommen werden.

Nitrierte Verbindungen können zu den entsprechenden Aminoverbindungen reduziert und die Aminoverbindungen gewünschtenfalls mit Alkyl halogeniden umgesetzt werden.

Die Nitrierung erfolgt ebenfalls nach an sich bekannten Methoden. Hierzu wird das Ausgangsmaterial bei Temperaturen unterhalb Raumtemperatur mit konzentrierter Salpetersäure umgesetzt. Konzentrierte Salpetersäure bedeutet die handelsübliche Form, die aber auch durch sogenannte rauchende Salpetersäure angereichert sein kann. Die Säure ist bei der Nitrierung sowohl Reaktionsmittel als auch Lösungsmittel.

Die sich gegebenenfalls anschließende Reduktion der erhaltenen Nitroverbindung zur entsprechenden Aminoverbindung erfolgt gleichfalls nach an sich bekannten Methoden.

Eine bevorzugte Methode ist die Reduktion mit Wasserstoff in Gegenwart von Metallkatalysatoren wie Raney-Nickel, Platin in feinverteilter Form oder Palladium auf einem geeigneten Träger wie Kohle

oder Kalk bei Normaldruck und Raumtemperatur. Möglich ist aber auch die Verwendung von Wasserstoff zu statu nascendi, z. B. durch Zink/Salzsäure.

Die Herstellung von Sulfonsäuren- oder Sulfonsäurederivaten erfolgt nach an sich bekannten Methoden Hierzu wird das Ausgangsmaterial in einem inerten Lösungsmittel wie Methylenchlorid, Chloroform gelöst, und unter Kühlung wird Chlorsulfonsäure zugesetzt.

Zur Herstellung von entsprechenden Alkylaminsulfonsäure-Derivaten wird das zuvor so erhaltene Produkt mit einem Alkylamin zur Reaktion gebracht.

Die Umsetzung von Indolderivat und Azadien nach Verfahrensvariante c) erfolgt in Gegenwart von Säuren bei Temperaturen zwischen 50 und 200 °C, vorzugsweise 75 bis 150 °C. Die Umsetzung wird zum Beispiel derart ausgeführt, daß das Indolderivat der Formel X und das Azabutadien der Formel XI in organischen Säuren, wie zum Beispiel Ameisensäure, Essigsäure, Propionsäure oder Trifluoressigsäure, oder im anorganischen Milieu, wie zum Beispiel Phosphorsäure, Polyphosphorsäure oder Phosphoroxychlorid usw. erhitzt werden. Es können auch inerte organische Lösungsmittel, wie zum Beispiel Toluol, Ethylacetat, Dioxan, Dimethoxyethan, Acetonitril u. a. als Verdünnungsmittel verwendet werden.

Zur Umsetzung können aber auch katalytische Mengen von Mineralsäuren wie Schwefelsäure, Salzsäure, Perchlorsäure etc. in inerten Lösungsmitteln (wie oben) verwendet werden.

Die Umsetzung ist nach mehreren Stunden abgeschlossen. Der Verlauf der Reaktion kann zum Beispiel durch Dünnschichtchromatographie verfolgt werden. Wenn das Ausgangsmaterial nach etwa 3 bis 10 Stunden umgesetzt ist, wird das Reaktionsgemisch in üblicher Weise aufgearbeitet.

Die als Ausgangsmaterial benötigten substituierten β-Carbolin-3-carbonsäureester können in bekannter Weise durch Aromatisierung entsprechend substituierter Tetra-bzw. Dihydro-β-carbolin-3-carbonsäureester erhalten werden, die ihrerseits durch Pictet-Spengler- bzw. Bischler-Napieralski-Cyclisierungen aus entsprechend substituierten Tryptophanestern zugänglich sind.

Vorteilhafter ist die Cyclisierung geeigneter ungesättigter Tryptophanderivate, wie zum Beispiel von α-Isocyano-indol-3-acrylestern, die in einer Stufe direkt die gewünschten β-Carbolin-3-carbonsäureester geben.

Besonders einfach ist jedoch die neuartige und überraschende regiospezifische Reaktion von gegebenenfalls substituierten Indolen mit 2-Azabutadien-3-derivaten gemäß Verfahrensvariante c), die in hohen Ausbeuten in einer Stufe die gegebenenfalls substituierten β-Carbolin-3-derivate liefert. Dieses neuartige Verfahren ist nicht auf die Synthese von β-Carbolin-3-carbonsäureestern beschränkt, sondern kann bei Einsatz von in 3-Position unterschiedlich substituierten 2-Azadienen (z. B., 3-Sulfonyl, 3-Dialkoxyphosphonyl) für die einstufige Synthese von den entsprechenden β-Carbolin-3-derivaten aus Indolen eingesetzt werden.

## Experimenteller Teil

### Herstellung von 3-Hydroxymethyl-β-carbolinen

A) 4,3 g β-Carbolin-3-carbonsäureethylester werden in 200 ml Tetrahydrofuran bei 4 °C unter Argon mit 900 mg Lithiumalanat portionsweise versetzt. Dann wird 30 Minuten bei Raumtemperatur und anschließend 1,5 Stunden bei 80 °C Badtemperatur gerührt. Darauf tropft man unter Eiskühlung 30 ml einer ln wässrigen NaOH Lösung zu, saugt vom Niederschlag ab, engt das Filtrat etwas ein und saugt die ausgefallenen Kristalle ab. Man erhält 3,15 g 3-Hydroxymethyl-β-carbolin vom Schmelzpunkt 230 °C.

Der β-Carbolin-3-carbonsäureethylester kann auf folgende Weise hergestellt werden :

a) 2 g α-Isocyano-indolyl-3-acrylsäureethylester und 200 mg 1.4-Diazabicyclo [2.2.2] octan werden in 200 ml Xylol 18 Stunden unter Stickstoff zum Sieden erhitzt. Nach dem Eindampfen und Kristallisation aus Ethanol oder Acetonitril erhält man 1,7 g β-Carbolin-3-carbonsäureethylester vom Schmelzpunkt 239 °C.

b) 1,2 g Indol und 3,2 g 3-Dimethylamino-2-(dimethylaminomethylenamino-acrylsäure-ethylester (hergestellt nach W. Kantlehner et al., Liebigs Ann. Chem. 1980, 344 A)) werden unter Stickstoff in 17 ml Eisessig gelöst und solange unter Rückfluß erhitzt, bis im Dünnschichtchromatogramm kein Ausgangsindol mehr sichtbar ist (6 Stunden). Nach Abdestillieren des größten Teil des Lösungsmittels wird in Wasser gegossen und das Kristallisat abgesaugt. Nach Umkristallisieren aus Acetonitril werden 1,45 g der Titelverbindung vom Schmelzpunkt 234 °C erhalten.

B) In analoger Weise werden aus den entsprechenden Estern hergestellt :

6-Brom-3-hydroxymethyl-β-carbolin, Schmelzpunkt 285-290 °C ;
6-Jod-3-hydroxymethyl-β-carbolin ;
4-Methyl-3-hydroxymethyl-β-carbolin, Schmelzpunkt 219-225 °C (Essigester/Ethanol) ;
4-Propyl-3-hydroxymethyl-β-carbolin, Schmelzpunkt 180-182 °C ;
6-Amino-3-hydroxymethyl-β-carbolin, Schmelzpunkt > 220 °C ;

5-Benzyloxy-4-methoxymethyl-3-hydroxymethyl-β-carbolin, Schmelzpunkt 188-190 °C (Essigester/Methanol) ;
6-Chlor-3-hydroxymethyl-β-carbolin, Schmelzpunkt 285-297 °C (Tetrahydrofuran) ;

C) Herstellung von 6-Acetylamino-3-hydroxymethyl-β-carbolinen

106 mg 6-Amino-3-hydroxymethyl-β-carbolin werden in 3 ml Eisessig mit 52 mg Essigsäureanhydrid bei Raumtemperatur 1,5 Stunden gerührt. Nach Filtrieren und Einengen wird aus Ethanol/Cyclohexan umkristallisiert und man erhält 24 mg 6-Acetylamino-3-hydroxymethyl-β-carbolin.

Beispiel 1

6-Jod-3-methylsulfinyl-β-carbolin

1,07 g 3-Methylthio-β-carbolin werden in 10 ml Eisessig mit 0,24 ml Wasser, 0,06 ml konzentrierter Schwefelsäure, 0,172 g Jodsäure und 0,442 g Jod bei 80 °C Badtemperatur 4 Stunden gerührt. Nach Zugabe von 172 mg Jodsäure und 442 mg Jod wird nochmals 3 Stunden auf 80 °C erhitzt. Nach Absaugen der ausgefallenen Kristalle erhält man 0,7 g 6-Jod-3-methylsulfinyl-β-carbolin vom Schmelzpunkt 280-285 °C.

Beispiel 2

6-Benzyloxycarbonyl-3-methylsulfinyl-β-carbolin

1,3 g 6-Jod-3-methylsulfinyl-β-carbolin werden in 21 ml Benzylalkohol mit 0,96 ml Tributylamin und 38 mg Palladium (II) acetat unter 1 atm Kohlenmonoxid 4 Stunden bei 110 °C carbonyliert. Nach Abdestillieren des Benzylalkohols wird in 75 ml Methylenchlorid aufgenommen und einmal mit 50 ml Inwässriger Salzsäure, einmal mit gesättigter Bicarbonatlösung und einmal mit gesättigter Kochsalzlösung extrahiert. Die organische Phase wird getrocknet, filtriert und eingeengt und der Rückstand aus Essigester/Hexan umkristallisiert, wobei man 630 mg 6-Benzyloxycarbonyl-3-methylsulfinyl-β-carbolin vom Schmelzpunkt 220-223 °C erhält.

Beispiel 3

6-Ethoxycarbonyl-3-methylsulfinyl-β-carbolin

270 mg 6-Benzyloxycarbonyl-3-methylsulfinyl-β-carbolin werden mit einer aus 27 mg 80 %igem Natriumhydrid und 15 ml absolutem Ethanol bereiteten Alkoholatlösung 1,5 Stunden am Rückfluß gekocht. Nach Einengen wird in Essigester aufgenommen und abgesaugt. Man erhält 78 mg 6-Ethoxycarbonyl-3-methylsulfinyl-β-carbolin vom Schmelzpunkt 240-243 °C.

Beispiel 4

3-tert.-Butoxycarbonylamino-β-carbolin

0,25 g β-Carbolin-3-carbonsaüre werden in 4 ml Dimethylsulfoxid unter Erwärmen gelöst. Zur Lösung werden 10 ml tert.-Butanol, 0,25 ml Diphenylphosphorylazid und 0,17 ml Triethylamin gefügt. Das Reaktionsgemisch wird 15 Minuten am Rückfluß gekocht. Die beim Abkühlen ausfallenden Kristalle werden abfiltriert, vom Filtrat wird im Vakuum der tert.-Butanol abgedampft und der Rückstand mit 10 ml Wasser versetzt. Aus dem hierdurch entstehenden Niederschlag werdern durch Chromatographie an Kieselgel (Methylenchlorid mit 5 % Methanol) 0,025 g 3-tert.-Butoxycarbonylamino-β-carbolin vom Schmelzpunkt 185-190 °C (Methanol) erhalten.

Beispiel 5

3-Methoxycarbonylamino-β-carbolin

4 g β-Carbolin-3-carbonsäureazid werden in 250 ml Methanol 6 Stunden am Rückfluß gekocht. Dann wird die Lösung eingedampft und der Rückstand zweimal aus Essigester umkristallisiert. Man erhält 0,14 g 3-Methoxycarbonylamino-β-carbolin vom Schmelzpunkt 236-238 °C.

Das als Ausgangsmaterial benötigte β-Carbolin-3-carbonsäureazid wird folgendermaßen hergestellt :

Zur Lösung von 3,3 g β-Carbolin-3-carbonsäure in 85 ml Dimethylsulfoxid werden 3,4 g Diphenylphosphorylazid und 2,2 ml Triethylamin zugefügt. Die Lösung färbt sich vorübergehend violett und es fällt ein Niederschlag aus. Nach Stehenlassen über Nacht wird der Niederschlag abfiltriert und das Filtrat in 0,8 l Wasser eingerührt. Der Niederschlag wird abgesaugt und getrocknet. Schmelzpunkt 138 °C (Verpuffung).

Beispiel 6

3-Acetamino-β-carbolin

2 g 3-Amino-β-carbolin werden in 35 ml Pyridin aufgeschlämmt und mit 4 ml Acetanhydrid versetzt. Das Aminocarbolin löst sich unter leichter Erwärmung. Nach 20 Minuten wird die Lösung eingedampft. Der kristalline Rückstand wird mit Wasser gewaschen und aus Essigester umkristallisiert. Ausbeute : 1,5 g 3-Acetamino-β-carbolin-vom Schmelzpunkt 200-203 °C.

Beispiel 7

3-(I-Pyrrolyl)-β-carbolin

0,74 g 3-Aminocarbolin und 0,54 g 2,5-Dimethoxytetrahydrofuran werden in 10 ml Eisessig 15 Minuten auf dem Dampfbad erwärmt. Nach dem Erkalten werden die ausgefallenen Kristalle abgesaugt und an Silicagel mit einer Mischung aus 10 Teilen Methylenchlorid und einem Teil Ethanol chromatographiert.

Ausbeute : 0,12 g 3-(I-Pyrrolyl)-β-carbolin ; Schmelzpunkt 165-170 °C.

Beispiel 8

3-Piperidino-β-carbolin

549 mg 3-Amino-β-carbolin werden mit 1,5 g 1.5-Dibrompentan und 1 g Diazabicycloundecen in 25 ml Tetrahydrofuran und 5 ml absolutem Ethanol 4 Stunden am Rückfluß gekocht. Nach Eindampfen wird über Kieselgel mit Cyclohexan/Essigester = 1 : 1 als Elutionsmittel chromatographiert. Man erhält 50 mg 3-Piperidino-β-carbolin als Öl.

Beispiel 9

3-Hydroxy-β-carbolin

0,75 g 3-Amino-β-carbolin werden in 85 ml 6-molarer Schwefelsäure gelöst und bei 0 °C mit einer Lösung von 0,435 g Natriumnitrit in 10 ml Wasser versetzt. Das Reaktionsgemisch wird eine Stunde bei 0 °C gerührt und anschließend zu 320 ml siedender 2-molarer Schwefelsäure getropft und noch 20 Minuten am Rückfluß gekocht. Nach Stehenlassen über Nacht wird mit Natriumhydroxid neutralisiert und mit einem Gemisch aus 10 Teilen Essigester und einem Teil Ethanol ausgeschüttelt. Der Essigesterextrakt wird einmal mit Wasser gewaschen, getrocknet und eingedampft. Der Eindampfrückstand wird aus Dimethylformamid umkristallisiert. So werden 0,2 g 3-Hydroxy-β-carbolin erhalten.

Beispiel 10

3-Ethoxy-β-carbolin

Zu einer Suspension von 1 g 3-Amino-β-carbolin in 100 ml Ethanol und 2 ml konzentrierter Schwefelsäure wird 1 ml Isoamylnitrit gefügt. Das Gemisch wird eine Stunde bei Raumtemperatur gerührt und anschließend eine halbe Stunde am Rückfluß gekocht. Hierbei entsteht eine klare Lösung. Sie wird nach dem Erkalten in ein Gemisch aus 100 ml Wasser und 100 ml gesättigter Natriumbicarbonatlösung gegossen. Es wird dreimal mit je 100 ml Ether ausgeschüttelt und der Eindampfrückstand der vereinigten Etherextrakte chromatographiert (Silicagel, Methanol/Chloroform = 95 : 5) und aus Hexan umkristallisiert. Ausbeute : 0,3 g 3-Ethoxy-β-carbolin vom Schmelzpunkt 130-133 °C.

Beispiel 11

Analog Beispiel 10 wird das 3-Methoxy-β-carbolin vom Schmelzpunkt 184-189 °C hergestellt.

Beispiel 12

3-Propoxy-β-carbolin

184 mg 3-Amino-β-carbolin werden in 15 ml n-Propanol bei Raumtemperatur mit 0,5 ml i-Amylnitrit versetzt und 15 Minuten gerührt. Dann wird 4,5 Stunden auf 80 °C Badtemperatur erhitzt. Nach Einengen wird über Kieselgel mit Methylenchlorid/Aceton = 1 : als Elutionsmittel chromatographiert. Nach Einengen und Umkristallisation der entsprechenden Fraktionen aus Diisopropylether/Cyclohexan erhält man 120 mg 3-Propoxy-β-carbolin vom Schmelzpunkt 147-151 °C.

Beispiel 13

Nach dem in Beispiel 12 angegebenen Verfahren werden hergestellt:
3-Isopropoxy-β-carbolin (Petrolether ausgerührt);
3-Benzyloxy-β-carbolin, Schmelzpunkt 174-175 °C, (Diisopropylether/Cyclohexan);
3-Cyclohexyloxy-β-carbolin, Schmelzpunkt 138-139 °C, (Diisopropylether/Cyclohexan);
3-(1-Methoxyethoxy)-β-carbolin, Schmelzpunkt 83-85 °C, (Diisopropylether/Cyclohexan);
3-t.-Butoxy-β-carbolin, Schmelzpunkt 208-212 °C, (Diisopropylether/Cyclohexan);
3-Butoxy-β-carbolin, Schmelzpunkt 114-115 °C, (Cyclohexan);
3-Isopentoxy-β-carbolin, Schmelzpunkt 95 °C

Beispiel 14

3-Ethylthio-β-carbolin

732 mg 3-Amino-β-carbolin werden in 7 ml Diethyldisulfid auf 80 °C erwärmt und mit 1 ml Isoamylnitrit versetzt. Nach 0,5 Stunden Erwärmen auf 80 °C wird nochmals mit 1 ml Isoamylnitrit versetzt und 1,5 Stunden auf 80 °C erhitzt. Nach Abdestillieren wird der Rückstand zweimal über Kieselgel mit Essigester/Cyclohexan als Elutionsmittel chromatographiert. Nach Ausrühren der entsprechenden, eingedampften Fraktion mit Cyclohexan/Diisopropylether erhält man 206 mg 3-Ethylthio-β-carbolin vom Schmelzpunkt 132-133 °C.

Beispiel 15

In entsprechender Weise werden hergestellt:
4-Methyl-3-methylthio-β-carbolin vom Schmelzpunkt 183-185 °C (ausgerührt Diisopropylether/Cyclohexan);
3-Benzylthio-β-carbolin vom Schmelzpunkt 150 °C, (Essigester/Diisopropylether);
3-Methylthio-β-carbolin vom Schmelzpunkt 178-179 °C, (chromatographisch gereinigt);

Beispiel 16

6-Nitro-3-methylthio-β-carbolin

970 mg 3-Methylthio-β-carbolin werden mit 60 ml einer 65 %igen Salpetersäure 4 Stunden bei Raumtemperatur gerührt. Nach Eintragen des Ansatzes in Wasser wird abgesaugt und getrocknet. Man erhält 950 mg 6-Nitro-3-methylthio-β-carbolin vom Schmelzpunkt > 250 °C.

Beispiel 17

6-Amino-3-methylthio-β-carbolin

950 mg 6-Nitro-3-methylthio-β-carbolin werden in 80 ml Tetrahydrofuran und 80 ml Ethanol mit 200 mg Palladium/Kohle (10 %ig) 7,5 Stunden bei Raumtemperatur unter Wasserstoffnormaldruck hydriert. Nach Filtrieren und Einengen wird der Rückstand über Kieselgel mit Methylenchlorid/Aceton = 1 : 1 als Elutionsmittel chromatographiert. Man erhält 600 mg 6-Amino-3-methylthio-β-carbolin vom Schmelzpunkt 202 °C.

Beispiel 18

6-Dimethylaminosulfonyl-3-methylthio-β-carbolin und 6.8-Bis-(dimethylaminosulfonyl)-3-methylthio-β-carbolin

400 mg 3-Methylthio-β-carbolin werden bei 0 °C portionsweise zu 1 ml Chlorsulfonsäure gefügt. Nach 1 Stunde Rühren bei Raumtemperatur wird langsam auf Eis gegeben, abgesaugt und der Rückstand getrocknet und anschließend in 5 ml Thionylchlorid mit 2 Tropfen Dimethylformamid erwärmt. Nach Einengen erhält man 500 mg eines Gemisches von 6-Chlorsulfonyl-3-methylthio-β-carbolin und 6.8-Bis-(chlorsulfonyl)-3-methylthio-β-carbolin.

200 mg des erhaltenen Gemisches werden mit 10 ml einer eiskalten 40 %igen Dimethylaminlösung versetzt und 5 Minuten auf 100 °C erhitzt. Nach dem Abkühlen wird mit Wasser verdünnt und mit Essigester extrahiert. Nach Chromatographie über Kieselgel mit Methylenchlorid/Ethanol = 95 : 5 als Elutionsmittel erhält man 130 mg 6-Dimethylaminosulfonyl-3-methylthio-β-carbolin vom Schmelzpunkt 298-300 °C sowie 90 mg 6.8-Bis-(dimethylaminosulfonyl)-3-methylthio-β-carbolin vom Schmelzpunkt 249-265 °C.

Beispiel 19

β-Carbolin-3-phosphonsäure-diethylester

Eine Mischung von 0,24 g Indol und 0,83 g Azadien A (siehe unten) in 3,4 ml Essigsäure wird 3 Stunden auf 140 °C (Badtemperatur) erhitzt. Beim Eingießen der Lösung in Eiswasser kristallisieren 0,28 g β-Carbolin-3-phosphonsäure-diethylester vom Schmelzpunkt 196 °C aus.

Beispiel 20

5-Benzyloxy-β-carbolin-3-phosphonsäure-diethylester

Analog Beispiel 19 werden aus 0,45 g 4-Benzyloxy-indol 0,50 g der Titelverbindung vom Schmelzpunkt 188 °C (aus Diethylether/Benzin) erhalten.

Beispiel 21

β-Carbolin-3-phosphonsäure-monoethylester

Analog dem Beispiel 19 wird in Gegenwart katalytischer Mengen Schwefelsäure der Monoester vom Schmelzpunkt > 320 °C (Zersetzung) erhalten.

Beispiel 22

β-Carbolin-3-phosphonsäure-monoethyl-monomethylester

0,28 g des Monoethylesters aus Beispiel 21 werden in Methanol gelöst und mit überschüssiger ätherischer Diazomethanlösung über Nacht stehengelassen. Man erhält 0,21 g der Titelverbindung.

Das in den Beispielen 19-21 verwendete Azadien wird folgendermaßen hergestellt :

Azadien A :

2-Dimethylamino-1-(dimethylaminomethylenamino)-ethen-phosphonsäure-diethylester

Eine Mischung von 3,7 g Aminomethanphosphonsäurediethylester und 15 g des Aminalesters tert. Butoxy-N.N.N'.N'-tetramethylmethandiamin werden 6 Stunden auf ca. 160 °C erhitzt. Nach fraktionierter Destillation des Rückstandes im Kugelrohr bei 160-165 °C erhält man 4,2 g.

**Patentansprüche**

1. β-Carboline der allgemeinen Formel I

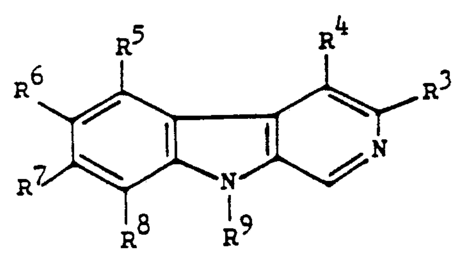

(I)

worin

$R^3$, $OR^I$ mit $R^I$ in der Bedeutung von H, $C_{1-5}$-Alkyl, $C_{3-7}$-Cycloalkyl, $C_{7-10}$-Aralkyl und $C_{5-10}$-Aryl, $NR^{II}R^{III}$ mit $R^{II}$ in der Bedeutung von H, $C_{1-5}$-Alkyl, $C_{3-7}$-Cycloalkyl, $C_{7-10}$-Aralkyl und $C_{5-10}$-Aryl und $R^{III}$ in der Bedeutung von $C_{1-3}$-Acyl, ($C_{1-6}$-Alkoxy)-carbonyl, Carbamoyl und $R_{II}$ und $R_{III}$ gemeinsam mit dem N-Atom ein gesättigter oder ungesättigter 5- oder 6-Ring, $SO_nR^I$, wobei n eine Zahl zwischen 0 und 2 ist und $R^I$ die oben angegebene Bedeutung hat, $PO_3R^{IV}R^V$ mit $R^{IV}$ und $R^V$ in der Bedeutung von H, $C_{1-5}$-Alkyl, $C_{3-7}$-Cycloalkyl, $C_{7-10}$-Aralkyl und $C_{5-10}$-Aryl, wobei $R^{IV}$ und $R^V$ gleich oder verschieden sein können,

$R^4$ H, $C_{1-5}$-Alkyl, Alkoxyalkyl, $COR^{VI}$ mit $R^{VI}$ in der Bedeutung von H, $C_{1-5}$-Alkyl, $C_{3-7}$-Cycloalkyl, $C_{7-10}$-Aralkyl, OH, O-$C_{1-5}$-Alkyl, O-$C_{3-7}$-Cycloalkyl, O-$C_{7-10}$-Aralkyl, $NR^{II}R^{II}$, wobei $R^{II}$ die oben angegebene Bedeutung hat und gleich oder verschieden sein können und mit dem N-Atom einen 5- oder 6-Ring bilden können, $CSR^{VII}$ mit $R^{VII}$ in der Bedeutung von H, $C_{1-5}$-Alkyl, $C_{3-7}$-Cycloalkyl und $C_{7-10}$-Aralkyl,

$R^5$-$R^8$ H, Halogen, $NO_2$, $OR^I$, $NR^{II}R^{III}$, $PO_3R^{IV}R^V$, $SO_2NR^IR^V$, $COOR^I$, $CONR^{II}R^{III}$, $CSNR^{II}R^{III}$ und $COR^I$, wobei $R^{I-V}$ die oben angegebene Bedeutung haben und

$R^9$ H, $C_{1-5}$-Alkyl, $C_{1-3}$-Acyl, Carbamoyl, ($C_{1-6}$-Alkoxy)-carbonyl und $SO_2R^{VIII}$ mit $R^{VIII}$ in der Bedeutung von Methyl und p-Tolyl,

bedeuten,

wobei der aromatische Ring mono- oder disubstituiert sein kann.

2. 6-Jod-3-methylsulfinyl-β-carbolin
6-Benzyloxycarbonyl-3-methylsulfinyl-β-carbolin
6-Ethoxycarbonyl-3-methylsulfinyl-β-carbolin

3. 3-tert.-Butoxycarbonylamino-β-carbolin
3-Methoxycarbonylamino-β-carbolin
3-Acetamino-β-carbolin
3-(1-Pyrrolyl)-β-carbolin
3-Piperidino-β-carbolin

4. 3-Hydroxy-β-carbolin
3-Ethoxy-β-carbolin
3-Methoxy-β-carbolin
3-Propoxy-β-carbolin
3-Isopropoxy-β-carbolin
3-Benzyloxy-β-carbolin
3-Cyclohexyloxy-β-carbolin
3-(1-Methoxyethoxy)-β-carbolin
3-tert.-Butoxy-β-carbolin
3-Butoxy-β-carbolin
3-Isopentoxy-β-carbolin
3-Ethylthio-β-carbolin
4-Methyl-3-methylthio-β-carbolin
3-Benzylthio-β-carbolin
3-Methylthio-β-carbolin
6-Nitro-3-methylthio-β-carbolin
6-Amino-3-methylthio-β-carbolin

5. 6-Dimethylaminosulfonyl-3-methylthio-β-carbolin
6.8-Bis(dimethylaminosulfonyl)-3-methylthio-β-carbolin
β-Carbolin-3-phosphonsäure-diethylester
5-Benzyloxy-β-carbolin-3-phosphonsäure-diethylester
β-Carbolin-3-phosphonsäure-monoethylester
β-Carbolin-3-phosphonsäure-monoethyl-monomethylester

6. Pharmazeutische Präparationen, gekennzeichnet durch den Gehalt an einer Verbindung der Ansprüche 1-5.

7. Verfahren zur Herstellung von Verbindungen der allgemeinen Formel I gemäß Anspruch 1, dadurch gekennzeichnet, daß man

a) eine Verbindung der Formel IV

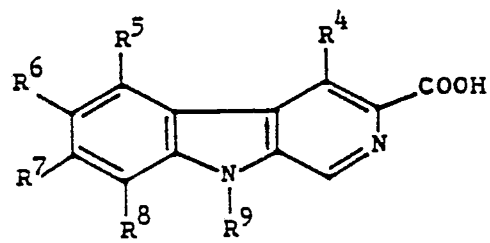

(IV)

worin $R^4$, $R^5$, $R^6$, $R^7$, $R^8$ oder $R^9$ die in Formel I angegebene Bedeutung haben, in an sich bekannter Weise zum 3-Carbonsäureazide umsetzt und das Carbonsäureazid nach Curtius in Gegenwart eines Alkohols der Formel $R^IOH$ thermisch zersetzt;

b) ein nach a) über 3-Carbonsäureazid durch Curtiusschen Abbau erhaltenes 3-Amino-β-carbolin der Formel VI

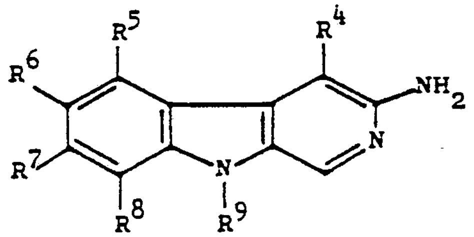

(VI)

worin $R^4$, $R^5$, $R^6$, $R^7$, $R^8$ und $R^9$ die oben angegebene Bedeutung haben, in an sich bekannter Weise acyliert, mit 2.5-Dimethoxy-tetrahydrofuran, 1.4-Dibromutan oder 1.5-Dibrompentan umsetzt oder in einer Sandmeyer-Reaktion mit verdünnter Schwefelsäure, mit Halogenwasserstoff in Gegenwart von Kupfer(I)-halogenid, mit Alkalimetallcyanid, mit $R^IOH$ oder $(R^I)_2S$, wobei $R^I$ die oben angegebene Bedeutung besitzt, umsetzt und gewünschtenfalls eine mit $(R^I)_2S$ erhaltene 3-$SR^I$-Verbindung zur 3-$SOR^I$- oder 3-$SO_2R^I$-Verbindung oxydiert und gewünschtenfalls die 3-$SR^I$- oder 3-$SOR^I$-Gruppe mit Raney-Nickel/Wasserstoff eliminiert und gegebenenfalls anschließend in 6-Stellung halogeniert und das so erhaltene Halogenierungsprodukt gegebenenfalls zur 6-$OR^I$- oder 6-$COOR^I$-Verbindung umsetzt und gegebenenfalls die 6-$COOR^I$-Verbindung umestert, verseift oder amidiert oder in 6-Stellung nitriert und gegebenenfalls die erhaltenen Nitroverbindungen zu den entsprechenden Aminoverbindungen reduziert und gegebenenfalls die Aminoverbindungen mit Alkyl halogenid umsetzt oder in 6-Stellung sulfonyliert und gegebenenfalls die so erhaltenen Chlorsulfonylverbindungen mit einem Amin umsetzt ;

c) ein Indol der allgemeinen Formel X

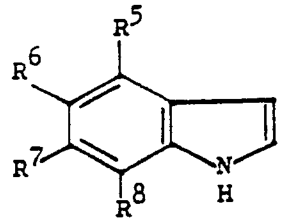

(X)

worin $R^5$, $R^6$, $R^7$ und $R^8$ die oben angegebene Bedeutung haben, mit einem Azadien der allgemeinen Formel XI

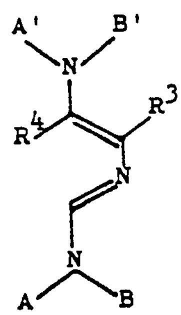

(XI)

worin $R^3$ und $R^4$ die oben angegebene Bedeutung haben und A und B sowie A' und B' jeweils für sich Alkyl mit 1-3 C-Atomen oder gemeinsam unter Einschluß von N eine Pyrrolidino-, Piperidino-, Morpholino- oder Piperazinogruppe bedeuten, in Gegenwart von Säuren bei Temperaturen von 50-200 °C umsetzt und gegebenenfalls einen in 3-Stellung erhaltenen Phosphonsäuremonoester in den Phosphonsäurediester überführt.

## Claims

1. β-Carbolines of the general formula I

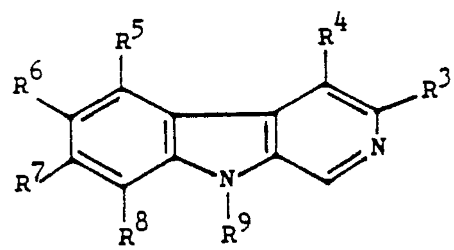

(I)

wherein

$R_3$ is $OR^I$ in which $R^I$ represents H, $C_{1-5}$-alkyl, $C_{3-7}$-cycloalkyl, $C_{7-10}$-aralkyl and $C_{5-10}$-aryl, $NR^{II}R^{III}$ in which $R^{II}$ represents H, $C_{1-5}$-alkyl, $C_{3-7}$-cycloalkyl, $C_{7-10}$-aralkyl and $C_{5-10}$-aryl and $R^{III}$ represents $C_{1-3}$-acyl, ($C_{1-6}$-alkoxy)-carbonyl, carbamoyl, and $R^{II}$ and $R^{III}$ together with the N atom form a saturated or unsaturated 5- or 6-membered ring, $SO_nR^I$ wherein n is a number between 0 and 2 and $R^I$ has the meanings given above, $PO_3R^{IV}R^V$ in which $R^{IV}$ and $R^V$ represent H, $C_{1-5}$-alkyl, $C_{3-7}$-cycloalkyl, $C_{7-10}$-aralkyl and $C_{5-10}$-aryl, wherein $R^{IV}$ and $R^V$ may be identical or different,

$R_4$ is H, $C_{1-5}$-alkyl, alkoxyalkyl, $COR^{VI}$ in which $R^{VI}$ represents H, $C_{1-5}$-alkyl, $C_{3-7}$-cycloalkyl, $C_{7-10}$-aralkyl, OH, O—$C_{1-5}$-alkyl, O—$C_{3-7}$-cycloalkyl, O—$C_{7-10}$-aralkyl, $NR^{II}R^{II}$ wherein $R^{II}$ has the meanings

given above and may be identical or different and may form with the N atom a 5- or 6-membered ring, $CSR^{VII}$ in which $R^{VII}$ represents H, $C_{1-5}$-alkyl, $C_{3-7}$-cycloalkyl and $C_{7-10}$-aralkyl,

$R^5$-$R^8$ is H, halogen, $NO_2$, $OR^I$, $NR^{II}R^{III}$, $PO_3R^{IV}R^V$, $SO_2NR^IR^V$, $COOR^I$, $CONR^{II}R^{III}$, $CSNR^{II}R^{III}$ and $COR^I$ wherein $R^{I-V}$ have the meanings given above, and

$R^9$ is H, $C_{1-5}$-alkyl, $C_{1-3}$-acyl, carbamoyl, ($C_{1-6}$-alkoxy)-carbonyl and $SO_2R^{VIII}$ in which $R^{VIII}$ represents methyl and p-tolyl,

it being possible for the aromatic ring to be mono- or di-substituted.

2. 6-Iodo-3-methylsulfinyl-β-carboline
6-Benzyloxycarbonyl-3-methylsulfinyl-β-carboline
6-Ethoxycarbonyl-3-methylsulfinyl-β-carboline

3. 3-tert.-Butoxycarbonylamino-β-carboline
3-Methoxycarbonylamino-β-carboline
3-Acetamino-β-carboline
3-(1-Pyrrolyl)-β-carboline
3-Piperidino-β-carboline

4. 3-Hydroxy-β-carboline
3-Ethoxy-β-carboline
3-Methoxy-β-carboline
3-Propoxy-β-carboline
3-Isopropoxy-β-carboline
3-Benzyloxy-β-carboline
3-Cyclohexyloxy-β-carboline
3-(1-Methoxyethoxy)-β-carboline
3-tert.-Butoxy-β-carboline
3-Butoxy-β-carboline
3-Isopentyloxy-β-carboline
3-Ethylthio-β-carboline
4-Methyl-3-methylthio-β-carboline
3-Benzylthio-β-carboline
3-Methylthio-β-carboline
6-Nitro-3-methylthio-β-carboline
6-Amino-3-methylthio-β-carboline

5. 6-Dimethylaminosulfonyl-3-methylthio-β-carboline
6,8-Bis(dimethylaminosulfonyl)-3-methylthio-β-carboline
β-Carboline-3-phosphonic acid diethyl ester
5-Benzyloxy-β-carboline-3-phosphonic acid diethyl ester
β-Carboline-3-phosphonic acid monoethyl ester
β-Carboline-3-phosphonic acid monoethyl monomethyl ester

6. Pharmaceutical preparations, characterised by a content of a compound of claims 1 to 5.

7. Process for the preparation of compounds of the general formula I according to claim 1, characterised in that

a) a compound of the formula IV

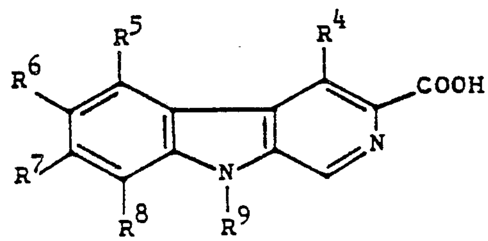

(IV)

wherein $R^4$, $R^5$, $R^6$, $R^7$, $R^8$ and $R^9$ have the meanings given for formula I, is reacted in a manner known per se to form the 3-carboxylic acid azide and the carboxylic acid azide is thermally decomposed in the presence of an alcohol of the formula $R^IOH$ according to a Curtius rearrangement;

b) a 3-amino-β-carboline obtained in accordance with a) via 3-carboxylic acid azide by Curtius decomposition and having the formula VI

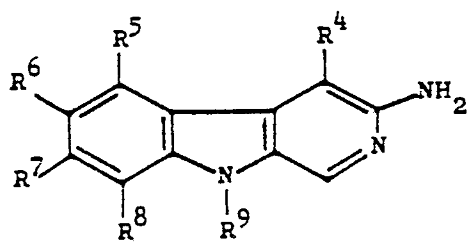

(VI)

wherein $R^4$, $R^5$, $R^6$, $R^7$, $R^8$ and $R^9$ have the meanings given above, is acylated in a manner known per se, reacted with 2,5-dimethoxytetrahydrofuran, 1,4-dibromobutane or 1,5-dibromopentane, or is reacted in a Sandmeyer reaction with dilute sulfuric acid, with hydrohalic acid in the presence of copper(I) halide, with alkali metal cyanide, with $R^IOH$ or $(R^I)_2S$ wherein $R^I$ has the meanings given above, and, if desired, a 3-$SR^I$ compound obtained with $(R^I)_2S$ is oxidised to a 3-$SOR^I$ or 3-$SO_2R^I$ compound and, if desired, the 3-$SR^I$ or 3-$SOR^I$ group is eliminated with Raney nickel/hydrogen and optionally halogenation in the 6-position is carried out, and the halogenation product so obtained is optionally reacted to the 6-$OR^I$ or 6-$COOR^I$ compound, and the 6-$COOR^I$ compound is optionally transesterified, hydrolysed or amidated or nitrated in the 6-position, and the resulting nitro compounds are optionally reduced to the corresponding amino compounds and the amino compounds are optionally reacted with alkyl halide or sulfonylated in the 6-position, and the chlorosulfonyl compounds so obtained are optionally reacted with an amine ;

c) an indole of the general formula X

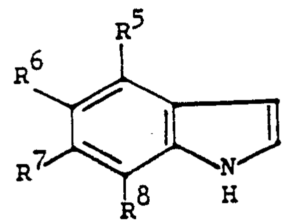

(X)

wherein $R^5$, $R^6$, $R^7$ and $R^8$ have the meanings given above is reacted in the presence of acids, at temperatures of from 50 to 200 °C, with an azadiene of the general formula XI

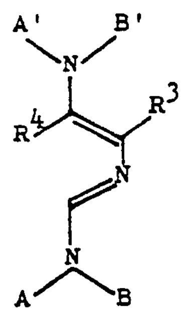

(XI)

wherein $R^3$ and $R^4$ have the meanings given above, and A and B and also A′ and B′ each represents alkyl having from 1 to 3 carbon atoms or together, including N, represent a pyrrolidino, piperidino, morpholino or piperazino group, and a phosphonic acid monoester obtained in the 3-position is optionally converted into the phosphonic acid diester.

## Revendications

1. β-Carbolines répondant à la formule générale I :

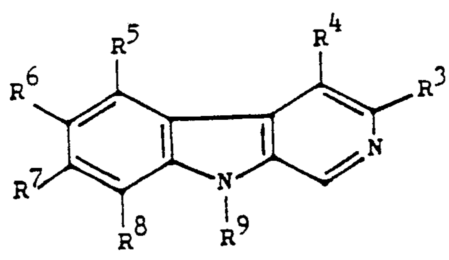

(I)

dans laquelle :

$R^3$ représente : un radical —$OR^I$ dans lequel $R^I$ représente H, un alkyle en $C_1$-$C_5$, un cycloalkyle en $C_3$-$C_7$, un aralkyle en $C_7$-$C_{10}$ ou un aryle en $C_5$-$C_{10}$, un radical —$NR^{II}R^{III}$ dans lequel $R^{II}$ représente H, un alkyle en $C_1$-$C_5$, un cycloalkyle en $C_3$-$C_7$, un aralkyle en $C_7$-$C_{10}$ ou un aryle en $C_5$-$C_{10}$ et $R^{III}$ représente un acyle en $C_1$-$C_3$, un alcoxycarbonyle à alcoxy en $C_1$-$C_6$ ou un carbamoyle, $R^{II}$ et $R^{III}$ pouvant aussi former ensemble, et avec l'atome d'azote, un cycle à 5 ou 6 maillons, saturé ou insaturé, un radical —$SO_nR^I$ dans lequel n désigne un nombre de 0 à 2 et $R^I$ a la signification qui lui a été donnée ci-dessus, ou un radical —$PO_3R^{IV}R^V$ dans lequel $R^{IV}$ et $R^V$, qui peuvent avoir la même signification ou des

significations différentes, représentent chacun H, un alkyle en $C_1$-$C_5$, un cycloalkyle en $C_3$-$C_7$, un aralkyle en $C_7$-$C_{10}$ ou un aryle en $C_5$-$C_{10}$,

$R^4$ représente H, un alkyle en $C_1$-$C_5$, un alcoxy-alkyle, un radical —$COR^{VI}$ (dans lequel $R^{VI}$ désigne H, un alkyle en $C_1$-$C_5$, un cycloalkyle en $C_3$-$C_7$, un aralkyle en $C_7$-$C_{10}$, —OH, un alcoxy en $C_1$-$C_5$, un cycloalcoxy en $C_3$-$C_7$, un aralcoxy en $C_7$-$C_{10}$ ou un radical —$NR^{II}R^{II}$ dont les symboles $R^{II}$ ont les significations précédemment données et peuvent être identiques ou différents, ou peuvent former, avec l'atome d'azote, un cycle à 5 ou 6 maillons), ou un radical —$CSR^{VII}$ dans lequel $R^{VII}$ représente H, un alkyle en $C_1$-$C_5$, un cycloalkyle en $C_3$-$C_7$ ou un aralkyle en $C_7$-$C_{10}$,

$R^5$ à $R^8$ représentent chacun l'hydrogène, un halogène, —$NO_2$ ou un radical —$OR^I$, —$NR^{II}R^{III}$, —$PO_3R^{IV}R^V$, —$SO_2NR^IR^V$, —$COOR^I$, —$CONR^{II}R^{III}$, —$CSNR^{II}R^{III}$ ou —$COR^I$, les symboles $R^I$ à $R^V$ ayant les significations indiquées ci-dessus, et

$R^9$ représente H, un alkyle en $C^1$-$C^5$, un acyle en $C^1$-$C^3$, un carbamoyle, un alcoxycarbonyle à alcoxy en $C_1$-$C_6$ ou un radical —$SO_2R^{VIII}$ dans lequel $R^{VIII}$ représente un radical méthyle ou p-tolyle,

et le noyau aromatique peut être mono ou disubstitué.

2. Iodo-6 méthylsulfinyl-3 β-carboline,
benzyloxycarbonyl-6 méthylsulfinyl-3 β-carboline et
éthoxycarbonyl-6 méthylsulfinyl-3 β-carboline.

3. tert-Butoxycarbonylamino-3 β-carboline,
méthoxycarbonylamino-3 β-carboline,
acétylamino-3 β-carboline,
(pyrrolyl-1)-3 β-carboline et
pipéridino-3 β-carboline.

4. Hydroxy-3 β-carboline,
éthoxy-3 β-carboline,
méthoxy-3 β-carboline,
propoxy-3 β-carboline,
isopropoxy-3 β-carboline,
benzyloxy-3 β-carboline,
cyclohexyloxy-3 β-carboline,
(méthoxy-1 éthoxy)-3 β-carboline,
tert-butoxy-3 β-carboline,
butoxy-3 β-carboline,
isopentoxy-3 β-carboline,
éthylthio-3 β-carboline,
méthyl-4 méthylthio-3 β-carboline,
benzylthio-3 β-carboline,
méthylthio-3 β-carboline,
nitro-6 méthylthio-3 β-carboline et
amino-6 méthylthio-3 β-carboline.

5. Diméthylaminosulfonyl-6 méthylthio-3
β-carboline,
bis-(diméthylaminosulfonyl)-6,8 méthylthio-3 β-carboline,
β-carboline-phosphonate-3 de diéthyle,
benzyloxy-5 β-carboline-phosphonate-3 de diéthyle,
β-carboline-phosphonate-3 de monoéthyle et
β-carboline-phosphonate-3 de monoéthyle et de monométhyle.

6. Compositions pharmaceutiques caractérisées en ce qu'elles contiennent un composé selon l'une quelconque des revendications 1 à 5.

7. Procédé pour préparer des composés de formule générale I selon la revendication 1, procédé caractérisé en ce que :

a) on fait réagir, de manière connue, un composé répondant à la formule IV :

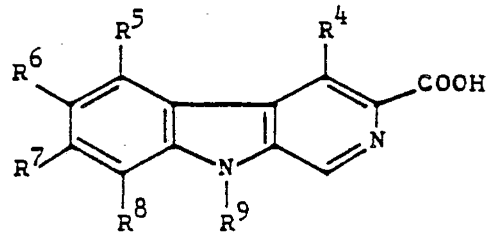

(IV)

dans laquelle $R^4$, $R^5$, $R^6$, $R^7$, $R^8$ et $R^9$ ont les significations qui leur ont été données à propos de la formule I, de manière à obtenir le composé à radical azidocarbonyle en 3, et on décompose thermiquement le composé azidocarbonylique par la méthode de Curtius en présence d'un alcool de formule $R^IOH$ ;

b) on acyle, de manière connue, une amino-3 β-carboline répondant à la formule VI (obtenue par une dégradation du Curtius passant par le composé à azidocarbonyle en 3) :

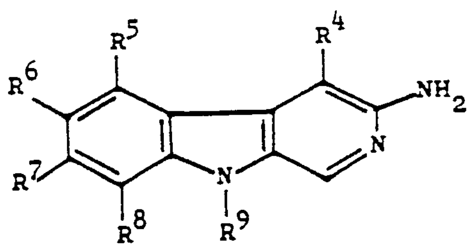

(VI)

dans laquelle $R^4$, $R^5$, $R^6$, $R^7$, $R^8$ et $R^9$ ont les significations qui leur ont été données ci-dessus, on fait réagir avec le diméthoxy-2,5 tétrahydrofuranne, le dibromo-1,4 butane ou le dibromo-1,5 pentane, ou on fait réagir, par la méthode de Sandmeyer, avec de l'acide sulfurique dilué, avec un halogénure d'hydrogène en présence d'un halogénure de cuivre(I), avec un cyanure de métal alcalin, avec $R^IOH$ ou $(R^I)_2S$ (le symbole $R^I$ a la signification qui lui a été donnée ci-dessus), et éventuellement on oxyde un composé à radical $—SR^I$ en 3, qui a été obtenu avec $(R^I)_2S$, de manière à le convertir en le composé contenant un radical $—SOR^I$ ou $—SO_2R^I$ en position 3, et, si on le désire, on élimine le radical $—SR^I$ ou $—SOR^I$ de la position 3 au moyen d'hydrogène en présence de nickel de Raney, puis on halogène éventuellement en position 6 et on convertit éventuellement le produit d'halogénation ainsi obtenu en le composé à radical $—OR^I$ ou $—COOR^I$ en position 6, et éventuellement on transestérifie, saponifie ou amidifie le composé à radical $—COOR^I$ en position 6 ou on nitre en position 6 et éventuellement on réduit les composés nitrés obtenus en les composés aminés correspondants, et éventuellement on fait réagir les composés aminés avec un halogénure d'alkyle ou on sulfonyle en position 6 et éventuellement on fait réagir avec une amine le composé chlorosulfonylique ainsi obtenu ;

c) on fait réagir un indole répondant à la formule générale X

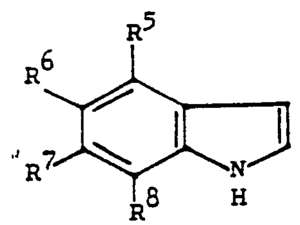

(X)

dans laquelle $R^5$, $R^6$, $R^7$ et $R^8$ ont les significations qui leur ont été données ci-dessus, avec un azadiène répondant à la formule générale XI

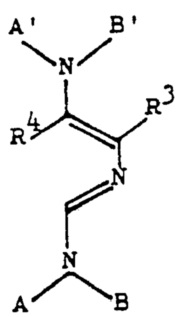

(XI)

dans laquelle $R^3$ et $R^4$ ont les significations qui leur ont été données ci-dessus et A et B, de même que A' et B', représentent chacun un alkyle en $C_1$-$C_3$ ou forment ensemble, et avec l'atome N, un radical pyrrolidino, pipéridino, morpholino ou pipérazino,
en présence d'acides, à des températures de 50 à 200 °C, et, si on a obtenu un mono-ester phosphonique à la position 3, on le transforme éventuellement en le diester phosphonique.